(19) Europäisches Patentamt
European Patent Office
Office européen des brevets

(11) Numéro de publication: **0 067 084**
**B1**

(12) **FASCICULE DE BREVET EUROPEEN**

(45) Date de publication du fascicule du brevet: **29.08.84**

(51) Int. Cl.³: **A 61 K 31/13**

(21) Numéro de dépôt: **82400843.7**

(22) Date de dépôt: **07.05.82**

(54) **Composition pour collyre destiné au traitement des conjonctivités et kératites.**

(30) Priorité: **04.06.81 FR 8111035**

(43) Date de publication de la demande: **15.12.82 Bulletin 82/50**

(45) Mention de la délivrance du brevet: **29.08.84 Bulletin 84/35**

(84) Etats contractants désignés: **AT BE CH DE GB IT LI LU NL SE**

(56) Documents cités:
**DE - A - 2 252 487**
**FR - A - 2 102 116**
**US - A - 3 291 683**

**Le dossier contient des informations techniques présentées postérieurement au dépôt de la demande et ne figurant pas dans le présent fascicule.**

(73) Titulaire: **Laboratoires P.O.S. Société Anonyme dite:, 21 rte de Lapoutroie, F-68240 Kaysersberg (FR)**

(72) Inventeur: **Andermann, Guy, 52, rue des Jonquilles, F-68000 Colmar (FR)**
Inventeur: **Andermann, Claudine, 52, rue des Jonquilles, F-66800 Colmar (FR)**

(74) Mandataire: **Cuer, André, CABINET CUER 30, rue de Léningrad, F-75008 Paris (FR)**

**EP 0 067 084 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention a trait au domaine des compositions pour collyres destinés au traitement des maladies de l'oeil. Elle concerne tout particulièrement un nouveau collyre antiseptique, destiné à être comme bactéricide d'action rapide dans diverses affections oculaires.

Divers agents actifs ont été préconisés et/ou utilisés comme antiseptiques dans de compositions pour collyres tels que par exemple: des dérivés mercuriels (type: thimérosal), des ammonium quaternaires (type cethexonium), des sulfamides (comme sulfacétamide), divers colorants (bleu de méthylène . . etc).

Il est toutefois difficile de sélectionner des ingrédients présentant à la fois une grande efficacité pour le traitement d'affections déterminées, une faible toxicité et une bonne tolérance biologique et clinique.

Il a maintenant eté trouvé que l'on pouvait mettre à la disposition des malades un collyre antiseptique très efficace, bien toléré et non toxique, spécialement apte à traiter les affections oculaires des types: conjonctivites bactériennes, blépharo-conjonctivites, kératites ponctuées superficielles, kératites métaherpétiques et maladies analogues.

Conformément à l'invention, la nouvelle composition pour collyre renferme essentiellement comme constituant actif l'amino-1 dodécyloxy-3 propane qui, par souci de simplification, sera dénommé »Lopa« dans la suite de la description.

Ce produit, de formule générale:

$CH_3-(CH_2)_{11}-O-(CH_2)_3-NH_2$ et poids moléculaire: 243,4

se présente sous la forme d'un liquide visqueux unicolore et limpide, insoluble dans l'eau, soluble dans divers solvants organique tels que: méthanol, chloroforme, acétone, benzène. A la connaissance de la Demanderesse, il n'a jamais été proposé ou utilisé comme produit actif de médicament et, dans l'application considérée ici, il doit avoir une pureté au moins 98% et présenter une perte à la dessication inférieure à 10% ainsi qu'un taux de cendres sulfuriques inférieur à 0,5%.

Outre le constituant actif susvisé, les compositions pour collyres selon l'invention peuvent renfermer divers autres constituants à fonctions diverses et jouant notammant les rôles: d'agents isotoniques, comme par exemple de l'acide borique; d'agents tampons, comme par exemple du chlorure de sodium et/ou du borate de sodium; éventuellement d'agents conservateurs comme par exemple des sels de mercure non toxiques. Bien entendu, comme dans le cas des autres collyres, les compositions sont diluées par de l'eau distillée.

Les quantités de »Lopa« mises en oeuvre dans le collyre selon l'invention peuvent varier entre d'assez larges limites, généralement entre 0,1 et 2 g/litre. Elles sont toutefois avantageusement maintenues entre les fourchettes de 0,10 et 1 g/litre. Quant aux proportions des adjuvants annexes, elles ne sont évidemment pas critiques et sont essentiellement fonction du type de produit utilisé. Un exemple de composition sera illustré au cours de la description suivante.

Des séries d'expériences et observations faites sur des collyres selon l'invention dans les domaines: toxicologie, pharmacologie, thérapeutique, ont donné des résultants extrêmement intéressants.

Au plan de la toxicité, les expériences d'administration par voie orale et par voie intrapéritonéale à des souris et à des rats ont montré une absence totale de toxicité aigü et une dose léthale DL 50 nettement supérieure aux doses maximales d'administration. Le test cytogénétique de mutagénèse (ou test micronucleus) s'est avéré négatif chez le rat. En outre, l'indice d'irritation oculaire est pratiquement nul chez le lapin et l'on a pu conclure à une très bonne tolérance dans l'oeil de l'homme.

Dans le domaine pharmacologique, les résultats d'expérimentations sur le lapin ont été hautement significatifs. Les essais de mesure de degré d'allergénicité (test de Magnusson), effectués sur cobayes, ont montré qu'il pouvait cependant se produire certains accidents allergiques dans quelques cas.

Par ailleurs les expériences d'activité mutagène sur diverses bactéries ont permis de conclure à l'absence de pouvoir mutagène du collyre conforme à l'invention.

Enfin, les séries de nombreuses études cliniques sur malades, entreprises en divers centres hospitaliers par des experts cliniciens en ophtalmologie, ont mis en évidence la grande efficaté du médicament pour de nombreuses affections, avec nette supériorité par rapport à des collyres connus de référence á bon pouvoir antiseptique.

L'invention sera mieux comprise par une description détaillée des essais et observations entrepris à partir d'un exemple, non limitatif, de réalisation d'une composition de collyre selon l'invention.

## Exemple de réalisation et expérimentation

Le collyre retenu pour la plupart des essais avait la composition suivante:

| | |
|---|---|
| Amino-1 dodécycloxy-3 propane (»Lopa«) | 0,025 g |
| Acide borique | 0,9 g |
| Borate de sodium | 0,14 g |
| Chlorure de sodium | 0,40 g |
| Eau purifiée, quantité suffisante pour | 100 ml |

Le produit actif Lopa avait une pureté d'au moins 98%.

a) Recherche de la dose léthale 50 (DL 50) chez la souris et le rat

Les études ont été menées d'une part sur un lot de 100 rats (type Sprague-Dawley), également répartis entre mâles et femelles et, d'autre part, sur le même nombre et la même répartition de souris (souche Swiss). Après une semaine d'acclimatation, on a administré aux animaux le collyre précité, soit par voie orale en utilisant un collyre en solution à 10% dans un »tween« (ester de sorbitanne) et avec des doses de 0,25 à 1,25 ml/100 g correspondant à despoids de 250 à 1 250 mg/Kg d'animal (exemple pour les rats), soit par voic intrapéritonéale en diluant le collyre dans du sérum physiologique et en administrant 0,5 ml de produit par 100 g de poids corporel pour le rat ou 0,1 ml/100 g pour la souris.

Bien entendu, on a noté quotidiennement le comportement, l'aspect des animaux, l'apparition éventuelle d'effets secondaires ainsi que la mortalité. Ceci pendant 14 jours après l'administration du produit.

On a pu ainsi déterminer que chez le rat la DL 50 était en moyenne de 720 mg/Kg pour la voie orale et de 55 mg/Kg pour la voie intrapéritonéale, alors que pour la souris ces DL 50 étaient respectivement de 1 193 mg/Kg et 63 mg/Kg en moyenne.

b) Test de Micronucleus

On sait que ce test, effectué in vivo (méthode SCHMID), est basé sur le fait que les cellules présentant des anomalies chromosomiques répartissent mal la chromatine au cours de mitose cellulaire provoquée par un produit toxique.

On a entrapris des séries d'essais en administrant le collyre précité, dilué à 10% (comme dans les expérimentations précédentes), chez des rats par voie orale en quantité de 1 mg/Kg. On a effectué des comparaisons d'une part avec des rats témoins, d'autre part sur des rats intoxiqués par l'aflatoxine $B_1$ (toxique cancérogène et mutagène expérimental connu). Les rats ont été sacrifiés après 30 heures de traitement et l'on a extrait et analysé la moelle osseuse pour déterminer l'activité cellulaire sous l'influence du produit mutagène. Ceci en examinant le pourcentage des micronucléi dans les Erythrocytes polychromatophiles ainsi que le rapport Erythrocytes polychromatophiles/Erythrocytes mûrs.

On a ainsi pu voir ce pourcentage et ce rapport étaient sensiblement voisins pour les animaux témoins et les animaux traités au collyre »Lopa« (% du Micronucleus : 0,2 à 0,4) alors que, pour l'aflatoxine, le % de Micronucleus était compris entre 8,8 et 10,6. On a pu conclure à l'absence totale de mutagénèse à la dose maximale de 1 g de collyre par Kg d'animal.

c) Essais de sensibilisation sur cobayes

Ces essais, effectués selon la technique connue de MAGNUSSON, sont destinés à rechercher les anomalies retardées consistant en des érythèmes, oedèmes, lésions eczémateuse qui sont caractéristiques de réactions allergiques cellulaires.

La sensibilisation a été ici étudiée par injection intradermiques du produit prétendu allergène en présence de l'adjuvant de Freund complet, suivies d'applications épicutanées de l'allergène.

Les essais ont été entrepris sur 10 cobayes de type Hartley pesant chacun 300 grammes et avec le collyre de l'invention soit sous forme d'émulsion pure soit sous de dilutions à 25% dans de la vaseline, ceci pour les applications épicutanées.

On a pu noter, à l'issue de ces expérimentations, l'absence de lésion eczémateuse. Toutefois certains animaux ont présenté une réaction épidermique avec rougeur faible ou moyenne diffuse, du type eczématiforme qui n'a guéri qu'après 14 jours suivant le test.

d) Essais de tolérance sur le lapin

Des séries d'expérimentations ont été effectuées sur des lots de lapins Albinos en pratiquant des instillations du collyre selon la composition précitée dans la paupière inférieure évasée de l'oeil droit, l'oeil gauche servant de témoin pour chaque lapin. Les animaux ont reçu 4 fois 4 gouttes par jour, à

intervalle de 2 heures et à raison de 6 jours de traitement par semaine.

Les études ont conduit aux résultats résumés ci-dessous: la mortalité à été nulle; la croissance pondérale était normale et constante; l'aspect et le comportement des animaux sont restés normaux dans tous les lots; le pouvoir irritant du collyre, déterminé selon l'indice d'irritation oculaire décrit dans le Journal Officiel (France) du 5/04/1971, était pratiquement nul (inférieur à 1 pour conjonctive, cornée et iris); l'examen ophtalmoscopique n'a pas montré de modification du fond de l'oeil en cours de traitement et aucune différence n'a pu être constatée entre l'oeil traité et l'oeil traité et l'oeil témoin. Enfin, les examens anatomopathologiques, effectués sur nerf oculaire, conjonctive et glandes annexes, n'ont montré aucune modification morphologique.

A la lumière de ces résultats expérimentaux, on a pu conclure que le collyre selon l'invention pouvait être administré chez l'homme aux doses conseillées par la Demenderesse.

e) Etude du pouvoir mutagène sur bactéries (Salmonella typhimurium)

On a testé à l'aide de différentes doses du collyre selon l'invention ($1 \cdot 10^{-3}$ à 2,5 mg par plaque) le système enzymatique hépatique provenant de rats pré-traités de type Sprague Dawley pesant 200 g et ayant reçu 5 jours avant leur sacrifice, une injection intrapéritonéale de 500 mg/Kg d'un inducteur enzymatique approprié, du type phénobarbital ou méthyl-chloranthrène.

Les séries d'essais ont montré que le collyre présentait un effet toxique pour toutes les souches de bactéries utilisées aux concentrations de 2,5 mg ou 0,5 mg par plaque avec et sans activation microsomiale et à la concentration de 0,125 mg par plaque sans activation microsomiale. Les résultats obtenus pour les autres concentrations ne montrent aucune différence du nombre de mutations pour les différentes souches examinées avec et sans activation microsomiale.

On a donc pu conclure à l'absence de pouvoir mutagène du collyre conforme à l'invention.

f) Essais de toxicité chronique

Des séries d'expériences d'une part sur le chien, après absorption orale d'une dose de 35 mg/Kg/jour pendant 13 semaines et, d'autre part, chez le lapin après instillation oculaire de 4 doses par jour d'une solution à 0,9 g (pour 100 ml) de Lopa pendant 13 semaines, ont montré qu'aucune toxicité locale et/ou systématique ne pouvait être décelée.

g) Etude de la bactériostase et de la bactéricide

La sensibilité du collyre Lopa selon l'invention a été étudiée sur 588 souches microbiennes isolées dans divers laboratoires d'analyses médicales et tirées de collections de divers Instituts. Pour cela on a utilisé la méthode des dilutions en eau purifiée stérile avec des concentrations décroissantes de 250 microgrammes/ml à 31,25 microgrammes/ml de collyre Lopa. La densité d'assemencement variait de $2 \cdot 10^{-3}$ à $10^{-4}$. La bactériostase (BS) a été appréciée par la concentration minimale inhibitrice lue après 48 heures pour les germes aérobies et après 96 heures pour les levures. La bactéricidie a été déterminée par passage d'un bouillon de caséine de soja, exempt de développement microbien, sur membrane filtrante et dépôt du bouillon sur gélose caséine-soja. Les principaux résultats obtenus sont rassemblés sur le tableau 3 ci-après où BS correspond à la bactériostase et BC à la bactéricidie.

Par ailleurs, divers facteurs susceptibles d'influencer l'activité bactéricide du collyre selon l'invention ont été étudiés sur plusieurs germes microbiens. Ainsi on a pu déterminer, par exemple, qu'à partir d'une concentration en germes de $10^5$, pour un temps de contact de 3 minutes et une concentration en collyre de 25 microgrammes, on ne décelait aucune germe sur Staphyloccus aureus et Escherichia Coli, alors que les germes envahissaient totalement la solution étalon.

D'autre part, pour cette même concentration en germes de $10^5$ et la même quantité de collyre (2 gouttes soit 25 microgrammes), des temps de contact de 1 minute sur Staphylococcus aureus et Escherichia Coli donnaient respectivement 75 germes et 10 germes, alors que la solution étalon était complètement envahie de germes. Le nombre de germes tombait à 0 pour le collyre de l'invention après un temps de contact de 3 minutes dans les deux cas précités.

h) Essais cliniques sur sujets humains

On a entrepris tout d'abord une étude de tolérance clinique et biologique du collyre de composition ci-dessus sur 20 malades dont 14 femmes et 6 hommes d'âge compris entre 3 et 86 ans et atteints de diverses affections dont: désinfection après décollement de la rétine, hyperhémie conjonctivale, désinfection de cataractes et glaucomes, conjonctivite, ulcère de cornée, trachome, uvéite .. Le collyre était instillé 4 à 5 fois par jour pendant des temps variables de 3 à 40 jours. Les tolérances

biologiques et cliniques ont toujours été bonnes ou excellentes.

Puis des séries d'essais ont été effectués d'une part sur 125 sujets de race blanche et d'autre part sur 72 sujets de race noire en opérant en double aveugle avec essais comparatifs au moyen d'un collyre de même composition que celui de l'invention mais où le »Lopa« avait été remplacé par du céthexonium, antiseptique connu pour les collyres.

On ne fournira pas ici les résultats analytiques détaillés tenant compte des différents groupes de malades avec les paramètres: sexe, âge, durée du traitement, etc. On se bornera à reproduire dans les tableaux 1 et 2 ci-après les résultats statistiques globaux sur les deux séries de malades de race blanche (tableau 1 et de race noire tableau 2) quant à l'effet thérapeutique au plan clinique:

Tableau 1

| Nbe malades | Résultats | | | |
|---|---|---|---|---|
| | très bons et bons | modérés | médiocres ou mauvais | total |
| traités par collyre de l'invention | 50 | 4 | 8 | 62 |
| traités par collyre au céthexonium | 36 | 8 | 18 | 62 |
| Total | 86 | 12 | 26 | 124 |

Tableau 2

| Nbe malades | Résultats | | | |
|---|---|---|---|---|
| | très bons et bons | modérés | médiocres ou mauvais | total |
| traités par collyre de l'invention | 24 | 0 | 8 | 32 |
| traités par collyre au céthexonium | 16 | 4 | 14 | 34 |
| Total | 40 | 4 | 22 | 66 |

Comme on peut le constater, le nombre de très bons et bons résultats est nettement supérieur dans le groupe de malades traités au collyre selon l'invention, par comparaison avec le groupe de malades traités au collyre de référence déjà connu. Le phénomène est inversé pour les résultats médiocres ou mauvais.

Des résultats du même type ont été obtenus au plan bactériologique à partir des prélèvements de germes effectués sur les malades.

En pratique, la posologie du traitement pour un collyre conforme à la composition de l'exemple précité est généralement de 4 à 6 instillations quotidiennes pendant des durées pouvant aller jusqu'à 30—40 jours sans inconvénient.

Parmi les nombreuses affections susceptibles d'être traitées par le collyre de l'invention on doit citer tout particulièrement: les conjonctivites bactériennes, les kératites ponctuées superficielles, les blépharo-conjonctivites, les kératites métaherpétiques, les conjonctivites catarrhales, etc.

Bien entendu, le principe actif du collyre selon l'invention (Lopa) peut également être mis en oeuvre sous forme de divers sels minéraux ou organiques tels que: chlorhydrate, oxalate, maléate, etc.

Tableau 3

| Nombre de souches étudiées | | Activité recherchée | Concentrations en µg/ml >250 | 250 | 125 | 62,5 | 31,25 |
|---|---|---|---|---|---|---|---|
| Staphylocoques non pathogènes | 85 | BS | | 7 | 78 | | |
| | | BC | | 7 | 78 | | |
| Staphylocoques pathogènes | 167 | BS | 9 | 45 | 90 | 23 | |
| | | BC | 9 | 45 | 90 | 23 | |
| E. Coli | 105 | BS | 18 | 41 | 46 | | |
| | | BC | 18 | 41 | 46 | | |
| Klebsielle | 17 | BS | 1 | 9 | 6 | | 1 |
| | | BC | 1 | 9 | 6 | | 1 |
| Proteus | 52 | BS | 13 | 12 | 27 | | |
| | | BC | 13 | 12 | 27 | | |
| Pseudomonas | 90 | BS | 28 | 41 | 20 | 1 | |
| | | BC | 28 | 41 | 20 | 1 | |
| Salmonella | 12 | BS | 4 | 7 | 1 | | |
| | | BC | 4 | 7 | 1 | | |
| Shigella | 3 | BS | | 1 | 2 | | |
| | | BC | | 1 | 2 | | |
| Streptocoque | 5 | BS | 1 | 2 | 2 | | |
| | | BC | 1 | 2 | 2 | | |
| Bacillus | 3 | BS | 1 | 1 | 1 | | |
| | | BC | 1 | 1 | 1 | | |
| Candida albicans | 49 | BS | 3 | 31 | 14 | 1 | |
| | | BC | 3 | 31 | 14 | 1 | |

**Revendications**

1. Collyre destiné au traitement d'affections oculaires telles que conjonctivites et kératites, caractérisé en ce qu'il renferme comme constituant actif de l'amino-1 dodécyloxy-3 propane sous forme de solution aqueuse à raison de 0,10 à 1 g/litre dans un agent tampon et isotonique du groupe acide borique et borate de sodium.

2. Collyre selon la revendication 1, caractérisé en ce que ladite solution aqueuse comprend en outre un produit conservateur.

3. Collyre selon l'une quelconque des revendications 1 ou 2, caractérisé en ce qu'il est constitué par une solution aqueuse comprenant;

| | |
|---|---|
| Amino-1 dodécyloxy-3 propane | 0,025 g |
| Acide borique | 0,9 g |
| Borate de sodium | 0,14 g |
| Chlorure de sodium | 0,40 |
| Eau purifiée, quantité pour | 100 ml |

**Patentansprüche**

1. Augentropfen bestimmt zur Behandlung von Augenleiden wie Konjunktivitis und Keratitis, dadurch gekennzeichnet, daß sie als Wirksubstanz 1-Amino-3-dodecyloxy-propan in Form einer wäßrigen Lösung von 0,10 bis 1 g/l in einer Puffersubstanz und einem isotonischen Mittel der Gruppe Borsäure und Natriumborat enthalten.

2. Augentropfen nach Anspruch 1, dadurch gekennzeichnet, daß die wäßrige Lösung außerdem ein

Konservierungsmittel enthält.

3. Augentropfen nach einem der Ansprüche 1 oder 2, dadurch gekennzeichnet, daß sie aus einer wäßrigen Lösung bestehen, die

| | |
|---|---|
| 1-Amino-3-dodecyloxy-propan | 0,025 g |
| Borsäure | 0,9 g |
| Natriumborat | 0,14 g |
| Natriumchlorid | 0,40 g |
| dest. Wasser, q.s.p. | 100 ml |

enthält.

**Claims**

1. Eye-lotion for ocular disenses such as cunjunctivites and keratites, characterized in that it contains as active principle 1-amino 3-dodecyloxy-propane under form of an aqueous solution of 0,10 to 1 g/liter in a buffering and isotonic agent selected of the groupe consisting off acid and sodium borate.

2. Eye-lotion according to claim 1, characterized in that the said aqueous solution contains also a preservative agent.

3. Aqueous eye-lotion composition according any of the claim 1 or 2, characterized in that it contains:

| | |
|---|---|
| 1-amino 3-dodecyloxy-propane | 0.025 g |
| Boric acid | 0.9 g |
| Sodium borate | 0.14 g |
| Sodium chloride | 0.40 g |

Purified water, sufficient to make up to100 ml